# EUROPEAN PATENT SPECIFICATION

(11) **EP 1 509 605 B1**
(45) Date of publication and mention of the grant of the patent: **30.09.2009**
(21) Application number: 03712468.2
(22) Date of filing: 15.04.2003
(51) Int. Cl.: C12N 15/12, C07K 14/705, A61K 38/17

(54) **PRODUCTION OF RECOMBINANT FRAGMENTS OF MUSCLE ACETYLCHOLINE RECEPTOR AND THEIR USE FOR EX VIVO IMMUNOADSORPTION OF ANTI-ACH RECEPTOR ANTIBODIES FROM MYASTHENIC PATIENTS**
PRODUKTION VON REKOMBINANTEN FRAGMENTEN AUS DEM MUSKEL-ACETYLCHOLINREZEPTOR UND IHRE VERWENDUNG FÜR EX VIVO IMMUNABSORPTION VON ANTI-ACH-REZEPTOR ANTIKÖRPER BEI PATIENTEN MIT MYASTHENIE
PRODUCTION DE FRAGMENTS DE RECOMBINAISON DU RECEPTEUR D'ACETYLCHOLINE MUSCULAIRE ET LEUR UTILISATION POUR UNE IMMUNO-ADSORPTION I EX VIVO /I D'ANTICORPS ANTI-RECEPTEURS DE L'ACETYLCHOLINE (ANTI-RACH) PROVENANT DE PATIENTS MYASTHENIQUES

(30) Priority: 17.04.2002 GR 2002100190
(43) Date of publication of application: 02.03.2005
(73) Proprietor: HELLENIC PASTEUR INSTITUTE, 115211 Athens (GR); ASSOCIATION FRANCAISE CONTRE LES MYOPATHIES, F-75013 Paris (FR); TZARTOS, Socrates, 161 21 Kaisariani Attikis (GR); MAMALAKI, Avgi, 106 83 Athens (GR)
(72) Inventor: TZARTOS, Socrates, GR-161 21 Kaisariani Attikis (GR); MAMALAKI, Avgi, GR-106 83 Athens (GR); PSARIDI-LINARDAKI, Loukia, GR-175 63 Paleo Faliro Attikis (GR); KOSTELIDOU, Kalliopi, GR-165 62 Glyfada Attikis (GR)
(74) Representative: Furlong, Isla Jane
(86) International application number: PCT/GR2003/000014
(87) International publication number: WO 2003/087373

(56) References cited:
- WO-A-97/17431
- WO-A-98/50544
- TZARTOS S J ET AL: "Expression of soluble ligand- and antibody-binding extracellular domains of human nicotinic acetylcholine receptor alpha1-subunit in yeast Pichia pastoris." SOCIETY FOR NEUROSCIENCE ABSTRACTS, vol. 27, no. 1, 2001, page 1278 XP001126983 31st Annual Meeting of the Society for Neuroscience;San Diego, California, USA; November 10-15, 2001 ISSN: 0190-5295
- YAO YUN ET AL: "Yeast expression and NMR analysis of the extracellular domain of muscle nicotinic acetylcholine receptor alpha subunit." JOURNAL OF BIOLOGICAL CHEMISTRY, vol. 277, no. 15, 12 April 2002 (2002-04-12), pages 12613-12621, XP002225375 April 12, 2002 ISSN: 0021-9258 cited in the application
- TAKAMORI M ET AL: "Immunoadsorption in myasthenia gravis based on specific ligands mimicking the immunogenic sites of the acetylcholine receptor." THERAPEUTIC APHERESIS: OFFICIAL JOURNAL OF THE INTERNATIONAL SOCIETY FOR APHERESIS AND THE JAPANESE SOCIETY FOR APHERESIS. UNITED STATES OCT 2001, vol. 5, no. 5, October 2001 (2001-10), pages 340-350, XP002225376 ISSN: 1091-6660 cited in the application
- D. BEESON ET AL: "Nucleotide sequence of human muscle acetylcholine receptor beta-subunit" NUCLEIC ACIDS RESEARCH., vol. 17, no. 11, 1989, page 4391 XP002268682 OXFORD UNIVERSITY PRESS, SURREY., GB ISSN: 0305-1048
- TAKAMORI MASAHARU ET AL: "Specific removal of antiacetylcholine receptor antibodies in patients with myasthenia gravis." TRANSFUSION SCIENCE, vol. 17, no. 3, 1996, pages 445-453, XP002225377 ISSN: 0955-3886 cited in the application
- TZARTOS SOCRATES J ET AL: "Anatomy of the antigenic structure of a large membrane autoantigen, the muscle-type nicotinic acetylcholine receptor." IMMUNOLOGICAL REVIEWS, vol. 163, June 1998 (1998-06), pages 89-120, XP008011767 ISSN: 0105-2896
- BEESON D ET AL: "PRIMARY STRUCTURE OF THE HUMAN MUSCLE ACETYLCHOLINE RECEPTOR CDNA CLONING OF THE GAMMA AND EPSILON SUBUNITS" EUROPEAN JOURNAL OF BIOCHEMISTRY, BERLIN, DE, vol. 215, no. 2, 1 July 1993 (1993-07-01), pages 229-238, XP000615672 ISSN: 0014-2956
- DAVID BEESON ET AL: "The human muscle nicotinic acetylcholine receptor alpha-subunit exists as two isoforms: a novel exon" EMBO JOURNAL, OXFORD UNIVERSITY PRESS, SURREY, GB, vol. 7, no. 9, 1 July 1990 (1990-07-01), pages 2101-2106, XP002077782 ISSN: 0261-4189
- TZARTOS S J ET AL: "SPECIFICITIES OF ANTIBODIES TO ACETYL CHOLINE RECEPTORS IN SERA FROM MYASTHENIA GRAVIS PATIENTS MEASURED BY MONO CLONAL ANTIBODIES" PROCEEDINGS OF THE NATIONAL ACADEMY OF SCIENCES OF THE UNITED STATES, vol. 79, no. 1, 1982, pages 188-192, XP002268683 1982 ISSN: 0027-8424
- JACOBSON LESLIE ET AL: "Monoclonal antibodies raised against human acetylcholine receptor bind to all five subunits of the fetal isoform" JOURNAL OF NEUROIMMUNOLOGY, vol. 98, no. 2, 3 August 1999 (1999-08-03), pages 112-120, XP002268684 ISSN: 0165-5728
- JANSEN K U ET AL: "EXPRESSION OF THE FOUR SUBUNITS OF THE TORPEDO-CALIFORNICA NICOTINIC ACETYLCHOLINE RECEPTOR IN SACCHAROMYCES-CEREVISIAE" JOURNAL OF BIOLOGICAL CHEMISTRY, vol. 264, no. 25, 5 September 1989 (1989-09-05), pages 15022-15027, XP002268685 ISSN: 0021-9258
- SHIBAHARA S ET AL: "CLONING AND SEQUENCE ANALYSIS OF HUMAN GENOMIC DNA ENCODING GAMMA SUBUNIT PRECURSOR OF MUSCLE ACETYLCHOLINE RECEPTOR" EUROPEAN JOURNAL OF BIOCHEMISTRY, BERLIN, DE, vol. 146, 1985, pages 15-22, XP002906423 ISSN: 0014-2956
- HINMAN C L ET AL: "IN-LINE AFFINITY CHROMATOGRAPHIC REMOVAL OF SPECIFIC ANTIBODY FROM RABBITS WITH EXPERIMENTAL MYASTHENIA GRAVIS AS A PRELUDE TO IMMUNOTHERAPY" IMMUNOPHARMACOLOGY AND IMMUNOTOXICOLOGY, MARCEL DEKKER, NEW YORK, NY, US, vol. 20, no. 2, May 1998 (1998-05), pages 233-249, XP008026933 ISSN: 0892-3973
- PSARIDI-LINARDAKI LOUKIA ET AL: "Expression of soluble ligand- and antibody-binding extracellular domain of human muscle acetylcholine receptor alpha subunit in yeast Pichia pastoris. Role of glycosylation in alpha-bungarotoxin binding." JOURNAL OF BIOLOGICAL CHEMISTRY, vol. 277, no. 30, 28 July 2002 (2002-07-28), pages 26980-26986, XP002225379 July 28, 2002 ISSN: 0021-9258
- PSARIDI-LINARDAKI LOUKIA ET AL: "Future therapeutic strategies in autoimmune myasthenia gravis." ANNALS OF THE NEW YORK ACADEMY OF SCIENCES. UNITED STATES SEP 2003, vol. 998, September 2003 (2003-09), pages 539-548, XP008026941 ISSN: 0077-8923

## Description

### Field of the invention

This invention relates to the field of extacorporeal blood processing, and more specifically to the antigen-specific elimination of autoantibodies for the treatment of autoimmune diseases.

### Background of the invention

Myasthenia gravis (MG) is a well characterized neurological autoimmune disease. It affects at least one out of 8.000 people. In MG, the characteristic weakness and fatiguability of the voluntary muscles result from antibody-mediated loss of the nicotinic acetylcholine receptors (AChR) at the neuromuscular junction. If swallowing and breathing muscles are involved, it can still be life-threatening. The pathogenic role of serum antibodies is clearly demonstrated by the dramatic clinical improvement that follows plasma exchange and by the passive transfer of experimental MG to animals by anti-AChR antibodies (Drachman, N. Eng. J. Med. 330,1797-1810, 1994; Vincent, Lancet, 357, 2122-2128, 2001). The AChR is a transmembrane glycoprotein consisting of five homologous subunits with the stoichiometry of alpha₂beta,gamma,delta or alpha₂beta,epsilon,delta (Devillers-Thiery et al, J. Membr. Biol. 3, 133-191, 1993; Karlin, Curr. Opin. Neurobiol. 3, 299-309, 1993). Effector mechanisms responsible for antibody-dependent AChR loss are: (a) cross-linking of AChRs by bivalent antibodies; (b) activation of complement; and probably less importantly (c) direct interference with the ion channel (Willcox, Curr. Opin. Immunol. 5, 910-917, 1993; Tzartos et al, Immunol. Rev. 163, 89-120, 1998). The mechanism that triggers the autoimmune response to the AChR is not known.

Current treatments of MG include anticholinesterase and immunosuppressive agents, thymectomy, plasmapheresis and intravenous human Ig (Massey, Neurology, 48, S46-S51, 1997). Although these therapies are often successful, they are non-specific and can be associated with severe side effects. Ideally, therapy should be antigen-specific and aimed at preventing the induction of or eliminating the specific antibodies.

Plasmapheresis is an efficient short-term treatment for MG, and clinical improvement correlates well with the reduction in circulating anti-AChR antibodies (Yeh et al, Acta Neurol. Scand., 100, 305-309, 1999). However, plasmapheresis is very expensive, because of the need to replace serum proteins, and removes non-pathogenic (protective) antibodies and other important molecules. An affinity column made of AChR or appropriate AChR fragments that bind selectively the anti-AChR antibodies would overcome many of these problems. Since native, and especially human, AChR is available only in very small quantities, specific and efficient immunoadsorption can only be achieved by the use of columns with recombinant AChR fragments. Because binding of the majority of the anti-AChR antibodies of MG patients is highly conformation dependent, denatured recombinant AChR fragments, like those usually obtained by procaryotic expression systems or synthetic peptides, are generally unable to bind significant fractions of the anti-AChR MG antibodies. Therefore, AChR fragments of near-native conformation are needed, and such may be preferably obtained by eucaryotic expression systems. Although most eucaryotic expression systems produce inappropriately low protein concentrations, there are a few systems which allow considerable production of recombinant proteins. These include the yeast *Pichia pastoris,* the Semliki Forest Virus (SFV) and the baculovirus expression systems.

Recombinant subunits, or their fragments, of non-human muscle AChRs have been produced in mammalian cells but in small amounts. These proteins bind some conformation dependent anti-AChR monoclonal antibodies but their ability to bind MG sera was not tested. Non-human AChR subunits have been also produced in Sf9 cells by the baculovirus expression system and in the yeast Saccharomyces expression system; no binding of MG antibodies was determined. Yao et al. (J. Biol. Chem. 227, 12613-12621, 2002) expressed in Pichia pastoris the extracellular domain of mouse AChR alpha-subunit (alpha1-211). No experiments were presented to show any binding of said molecule to MG antibodies, nor any speculation of its possible MG antibody binding capacity or of its use for immunoadsorption was made. In any case, it is known since many years that non-human AChRs are able to bind only small fractions of the MG antibodies (and therefore they are not appropriate for use as immunoadsorbents in MG). Thus, Lindstrom et al. (Muscle and Nerve 1,140-145, 1978) in 24 tested MG sera, found that reactivity of said sera with mouse AChR was only 0.3-15% (average 4.3%) of that with human AChR. Similarly low were the percentages for AChRs from other species. Also, Vincent & Newsom-Davis (Clin. Exp. Immunol. 49,257-265, 1982 and 49,266-272, 1982) in a similar study on 37 patients with generalized, 7 with ocular and 11 with drug-induced MG, they determined their cross-reactivity with mouse AChR as: 11.8%, 1.6% and 3.7% of that with human AChR, respectively. Our group too (Loutrari et al., Eur. J. Immunol. 22,2949-2956, 1992) in 10 MG sera found average cross-reactivity with mouse AChR: 15.3%. Several other observations in the past have shown the low cross-reactivity of MG antibodies with animal AChRs. Therefore, it is concluded that animal AChRs (with the possible exception of primate AChRs due to their higher similararity with human AChR), and even more the fragments/domains of said AChRs, are not appropriate tools for use as efficient immunoadsorbents of the anti-AChR antibodies from MG patients. For the same reason, the immunochemical diagnosis of MG is generally performed internationally by the use of human AChR rather than by the more easily available animal AChRs (e.g. Kalden et al., Autoimmunity, 1,59-66, 1988).

Recombinant extracellular domains of human alpha-subunit have been earlier produced in prokaryotic expression systems (E. coli). Barchan et al. (Eur. J. Immunol. 28, 616-624, 1998) produced in E. coli polypeptides of various lengths corresponding to parts or all of the extracellular domain of human AChR alpha-subunit. They showed that said polypeptides bind anti-AChR monoclonal antibodies, mainly those to conformation-independent epitopes, and that said polypeptides are capable of protecting mice against experimental MG induced by said anti-AChR monoclonal antibodies. No investigation was made regarding the possible MG antibody binding capacity of said polypeptides nor of their use for immunoadsorption. Barchan et al. (WOA9850544, 1998) also produced similar polypeptides and proposed their use, in addition to the experiments described in the above Barchan et al. paper, to modulate the autoimmune response of an individual to the AChR. Said approach is totally different from immunoadsorption, it consists of administering orally or nasally said polypeptides aiming at the deletion/anergy of antigen-specific T cells (therefore, no correct conformation of said polypeptides is required); no investigation was made regarding the possible MG antibody binding capacity of said polypeptides nor of their use for immunoadsorption. It was however suggested that expressions in eukaryotic cells would result in said alpha-subunit molecules with the right conformation. Tsouloufis et al. (Int. Immunol. 12, 1255-1265, 2000) produced in E. coli a polypeptide corresponding to the extracellular domain of human AChR alpha-subunit (alpha1-208), and achieved its partial refolding and its improved binding to anti-AChR monoclonal antibodies. No investigation was made regarding the possible binding of said polypeptide to antibodies from MG patients nor of the use of said polypeptide for immunoadsorption.

Immunoadsorbent columns with resin-linked tryptophan (TR 350^{@}), phenylalanine or protein A (Immunosorba.RTM, Excorim.RTM, Lund, Sweden) have been used in MG and in other autoimmune diseases. These columns immunoadsorb large fractions of the plasma IgG together with similar or larger fractions of anti-AChR antibodies. Yet, this procedure is non-specific and eliminates useful immunoglobulins from the plasma.

Takamori et al, (e.g. Transfus Sci. 17, 445-53, 1996; and Therapeutic Apheresis, 5, 340-350, 2001) and Tanihara et al. (US patent 5,132,402, 1992) describe columns (MG50^{@}, Medisorba MG) on which a small segment of Torpedo AChR alpha subunit (residues 183-200, i.e. 12 times smaller than the whole extracellular domain of a single subunit) which contains part of the ligand binding site of the AChR, produced chemically (not recombinantly), was permanently immobilized. Such columns were used as immunoadsorbents for MG treatment, yet the immunoadsorption efficiency of such columns is very limited. Said peptide represents a very small fraction of the extracellular surface of an AChR from a non mammalian species (the fish *Torpedo*). At best, the restricted aim of such an immobilized peptide can be to immunoadsorb some antibodies which bind to sites located near the ligand binding site of the AChR. In fact, the assay used by the authors for measuring the eliminated antibodies (AChR-blocking assay) measures the fraction of the antibodies which interfere with binding of alpha-bungarotoxin (alpha-BTX) to the AChR. Said alpha-BTX blocking antibodies do not necessarily block binding of the ligand acetylcholine (Gomez & Richman, J. Immunol. 135, 234-241, 1985) (since alpha-BTX is a protein molecule many times larger than acetylcholine). It is possible, however, though not determined in the above documents, that a smaller fraction within said antibody fraction may also block binding of acetylcholine and thus may block AChR function. Yet, such antibodies constitute a minority among the total anti-AChR antibodies in the MG sera, and their potential role in MG pathogenicity is, at best, one of the three known mechanisms by which anti-AChR antibodies act in MG pathogenicity (Vincent, Lancet, 357,2122-2128, 2001). Furthermore, the origin of the AChR used in the alpha-BTX blocking assays is not specified in the above documents, i.e. whether it was human or animal (in the later case, the detected blocking antibodies would have been an even smaller fraction of the MG patients' anti-AChR antibodies). The fraction of the eliminated overall AChR-binding antibodies was similarly low with the fraction of the eliminated total non-specific IgG (15%). Finally, the authors themselves recognized that the small peptide used is capable of removing "only the anti AChR blocking antibody, one set among the various anti-AChR antibodies implicated in the pathogenesis of myasthenia gravis" (page 451 of Takamori et al. 1996, and page 349 of Takamori et al. 2001).
Takamori et al. (Transfusion Science, vol. 17, no. 3, 1996, pages 445-453) discloses the use of a single synthetic AChR peptide derived from amino acids 183-200 of the alpha subunit of AChR as an immunoadsorbent.
WO 98/50544 discloses the use of recombinant fragments of the alpha subunit of AChR to modulate the autoimmune response of an individual to the AChR.
Jacobson et al. (J. Neuroimmunology 98 (1999) 112-120) uses a combination of Western blotting and immunoprecipitation to demonstrate the subunit specificity of monoclonal antibodies raised against purified AChR.

Our approach involves the production of large recombinant AChR fragments (more than 70 amino acids long, preferably around 200-220 amino acids long) which when permanently (i.e. covalently) immobilized on insoluble carriers are capable of eliminating the majority of the MG antibodies, for use as highly specific immunoadsorbents for the *ex vivo* selective elimination of the patients' anti-AChR antibodies. Since the great majority of the anti-AChR antibodies of MG patients bind extracellularly (Tzartos et al., Proc. Natl. Acad. Sci. USA 79, 188-192, 1982; Tzartos et al, Immunol. Rev. 163, 89-120, 1998), and especially since only said antibodies can be pathogenic, we are expressing only the extracellular parts of the human AChR subunits as being water-soluble proteins and therefore more conveniently used. However, expression of larger fragments or intact subunits containing the extracellular domains, or smaller fragments containing large parts of the extracellular domains of the AChR subunits, will apparently behave similarly and are covered by this patent application.

Production of the extracellular domain of human AChR alpha-subunit in yeast Pichia pastoris has been presented in the abstract book of a Conference (Tzartos et al., Society for Neuroscience Abstracts, vol. 27, No 1, p.1278, 2001), although no presentation of said work was made in the Conference. Said publication announces briefly the production of said polypeptide without disclosing the methodology used, and no information is presented on its capacity to bind large quantities of antibodies from MG patients (but that simply it binds MG antibodies), nor on its use as immunoadsorbent. Psaridi-Linardaki et al. (J. Biol. Chem. 277, 26980-26986, 2002) presented more details on the production of said alpha-subunit extracellular polypeptide and also revealed its capacity to bind large quantities of MG antibodies, although no immunoadsorption was presented. Said document was published later than our Priority Date claimed (date of submission of the Greek Application).

A generally efficient specific immunoadsorbent for MG necessitates the use of the extracellular domains from all or several of the human muscle AChR subunits. The produced and used polypeptides (of alpha, beta, gamma, delta and epsilon subunits) are complementary, since they are parts of a single protein molecule (the AChR), each one is capable of eliminating a different fraction of the pool of the anti-AChR antibodies from the MG patients, and their combined use is highly advantageous (usually necessary) since it allows the elimination of the majority of the anti-AChR antibodies from the majority of the MG patients. Therefore, production and use of said polypeptides (of alpha, beta, gamma, delta and epsilon subunits) constitutes a single invention with a single aim.

### Summary of the invention

According to a first aspect of the present invention there is provided use of a combination of recombinant N-terminal extracellular domains of the alpha, beta, gamma, delta and epsilon subunits of the human muscle nicotinic acetylcholine receptor (AChR) in an ex-vivo method of immunoadsorption of anti-AchR antibodies of myasthenia gravis (MG) patients.

According to another aspect of the present invention there is provided an insoluble carrier comprising a combination of recombinant N-terminal extracellular domains of the alpha, beta, gamma, delta and epsilon subunits of the human muscle nicotinic acetylcholine receptor (AChR) wherein the domains are covalently immobilized to the carrier matrix.

According to another aspect of the present invention there is provided a method for making an insoluble carrier for use in a method of immunoadsorption of anti-AChR antibodies comprising:
i) expressing a combination of recombinant N-terminal extracellular domains of the alpha, beta, gamma, delta and epsilon subunits of the human muscle nicotinic acetylcholine receptor (AChR);
ii) covalently immobilizing said purified domains onto an insoluble carrier matrix.

According to another aspect of the present invention there is provided a method for *ex vivo* removal of anti-AChR antibodies from the blood of MG patients comprising incubating said blood with an insoluble carrier of the present invention.

The N-terminal extracellular domains (for example amino adds 1-210/211, 1-222, 1-218, 1-224, 1-219 of the alpha, beta, gamma, delta and epsilon subunits respectively (as well as smaller fragments of 80-120 amino acids long) of the human muscle nicotinic acetylcholine receptor (AChR), are expressed in very efficient systems, preferably eucariotic systems, including the yeast *Pichia pastoris* with the purpose to be used as immunoadsorbents of anti-AChR antibodies of myasthenia gravis (MG) patients as a novel antigen-specific immunotherapy of said disease. Alpha1-210 binds ¹²⁵I-alpha-α-bungarotoxin with high affinity. Several conformation-dependent anti-AChR monoclonal antibodies are able to bind to these proteins as do antibodies from a large proportion of MG patients. The purified proteins are subsequently permanently (covalently) immobilized on insoluble carriers (for example Sepharose-CNBr) and are used to immunoadsorb anti-AChR antibodies from MG sera. In the immunoadsorbent columns that we propose, the alpha subunit fragment eliminates more than 60% (60-94%) of the anti-AChR antibodies in 20% of the sera, whereas on the average it eliminated 37% of the anti-AChR antibodies in all sera tested. A similar alpha-subunit fragment expressed in E. coli and immobilized on similar columns (and refolded) is also able to immunoadsorb MG antibodies but at a much lower level. Each of the extracellular fragments of the other subunits (beta, gamma, delta and epsilon) precipitated lower but very significant percentages of anti-AChR antibodies from said sera. Finally, extracellular polypeptides from different subunits, permanently immobilized on solid carriers, were mixed and used as immunoadsorbents. Said mixed immunoadsorbents eliminated increased percentages of the anti-AChR antibodies, approximately equal to the sum of the immunoadsorptions achieved by the individual subunit immunoadsorbents. Thus, although any single immobilized subunit polypeptide may be capable of eliminating large percentages of anti-AChR antibodies from a few MG patients, the combined use of said polypeptides is highly preferable for the therapeutic application in the majority of the MG patients.

### Brief description of the drawings

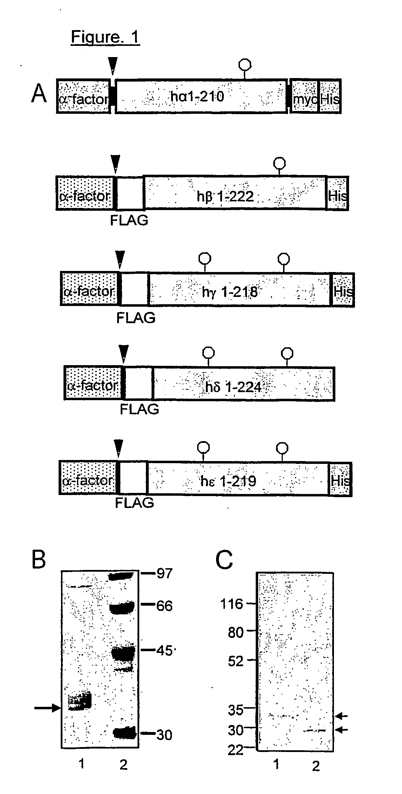

**Figure 1** illustrates the constructs used for expression of alpha1-210, beta1-222, gamma1-218, delta1-224 and epsilon1-219 subunit domains in yeast *Pichia pastoris* (A), and the glycosylated (B) and deglycosylated (C) forms of alpha1-210 protein. (A) All five subunit extracellular domains were led by a signal peptide (alpha-factor), which is cleaved by host enzymes after secretion (the filled triangle represents the cleavage site). Alpha1-210 was fused at the C-terminal end to a c-myc epitope and a six-histidine tag. All remaining four extracellular subunit domains carried the FLAG epitope just downstream the alpha-factor peptide and all except delta1-224 were fused at the C-terminus with a six-histidine tag. (B) Purification of alpha1-210 using NiNTA metal affinity chromatography: purified alpha1-210 was analyzed by 12 % SDS-PAGE (lane 1) and the proteins stained with Coomassie Brilliant Blue; the positions of the molecular weight markers are indicated (lane 2). The obtained results were similar for all remaining four subunit domains (beta, gamma, delta and epsilon). (C) Deglycosylation of alpha1-210 by peptide N-glycosidase F. Purified alpha1-210 was incubated at 37°C for 6 hours in the absence (lane 1) or presence (lane 2) of N-glycosidase F, then the mixture was analysed by 12% SDS-PAGE followed by western blotting using mAb 198. The arrows indicate the band corresponding to the glycosylated (upper) and deglycosylated (lower) forms of alpha1-210. All other subunits were also analysed for deglycosylation and in all cases, they were found to be expressed in glycosylated form like alpha1-210. Deglycosylation of each protein by enzyme N-glycosidase F led to the appearance of a deglycosylated form of the protein, corresponding to the predicted molecular weight.

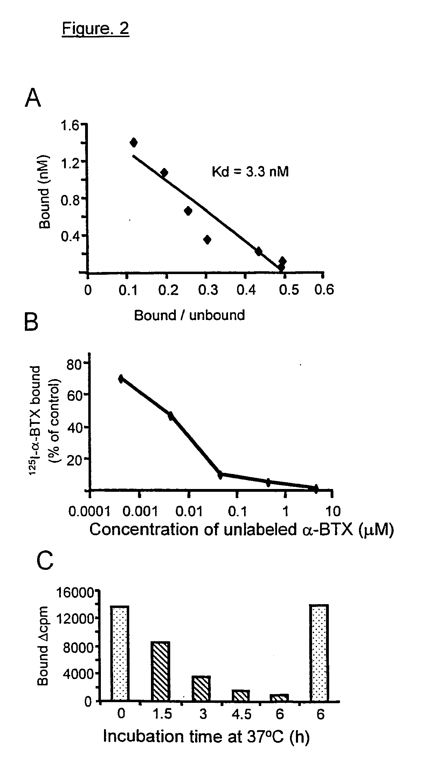

**Figure 2** illustrates the binding of alpha-BTX to alphal-210 of Fig. 1. (A) Scatchard plot analysis of ¹²⁵I-alpha-BTX binding to alpha1-210 fragment. Culture supernatants containing alphal-210 (40 microliters, 40 ul) were incubated with various concentrations of ¹²⁵I-alpha-BTX in 10 ul of PBS-0.2 % bovine serum albumin (BSA), then, after 3 hours incubation at 4° C, the samples were filtered through Whatman DE81 filters, and the bound radioactivity measured on a gamma counter (filter assay). Samples were processed in duplicates. The estimated Kd in the representative experiment shown is 3,3 nM while the average Kd of a series of experiments was 5.1 ± 2.4 nM. (B) The specific binding of the ¹²⁵I-alpha-BTX to alphal-210 fragment was studied in competition experiments involving the simultaneous addition of increasing concentrations of unlabeled toxin. Samples were processed in duplicates. Binding of labeled alpha-BTX in the absence of unlabeled alpha-BTX was taken as the 100% level. (C) Effect of deglycosylation on ¹²⁵I-alpha-BTX binding to alpha1-210. *In vitro* deglycosylation was performed using N-glycosidase F treatment. Aliquots at various reaction time-points were assayed for ¹²⁵I- alpha-BTX binding in filter assay experiments. Samples were processed in duplicates (dark columns, N-glycosidase F treatment; light columns, control treatment).

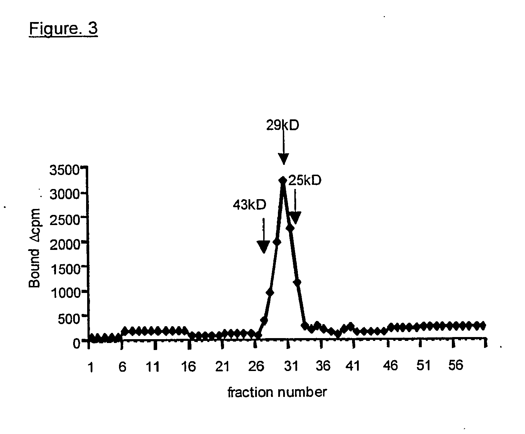

**Figure 3** illustrates FPLC analysis of alphal-210 of Fig. 1. FPLC-purified alpha1-210 has a molecular weight of ∼ 34 kD. Gel filtration fractions (Superose-12 column, PBS buffer, pH 7.4, flow rate 0.5 ml/min) were assayed for ¹²⁵I-alpha-BTX binding. The arrows indicate fractions corresponding to polypeptides of known molecular weight.

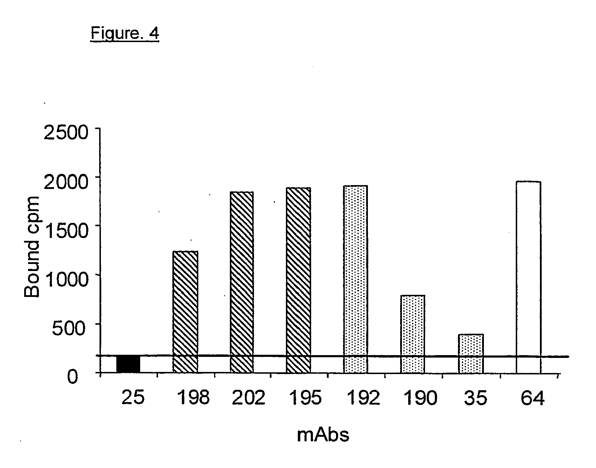

**Figure 4** illustrates the binding of conformation-dependent and partially conformation-dependent monoclonal antibodies (mAbs) to alpha1-210 fragment. Three groups of mAbs were used in radioimmunoassays (RIA): a. anti-MIR (main immunogenic region) mAbs, the binding of which is partially conformation-dependent (mAbs 198, 202, 195) (hatched columns), b. anti-MIR mAbs that bind only to native receptor (mAbs 192, 190, 35) (light columns), and c. mAb 64, an anti-alpha subunit mAb that does not bind to the MIR (blank column) and the binding of which is conformation-dependent. mAb 25 was used as a negative control (black column). Samples were processed in duplicates.

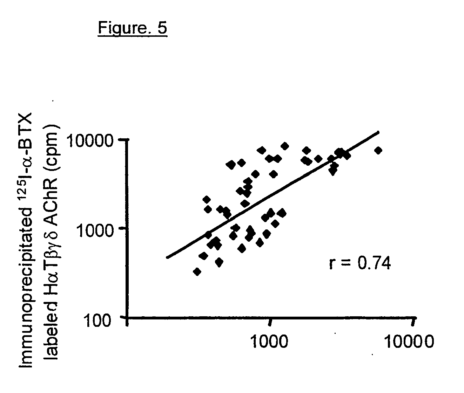

**Figure 5** illustrates the binding of anti-alpha autoantibodies from a high proportion of MG sera to recombinant alpha1-210. 50 MG-positive sera were assayed for binding to alpha1-210 and to a hybrid AChR molecule (HaTbgd) containing human alpha subunit and Torpedo beta, gamma and delta subunits, labeled with ¹²⁵I-alpha-BTX. MG sera binding to ¹²⁵I-alpha-BTX-alpha1-210 were plotted versus binding to ¹²⁵I-alpha-BTX-HaTbgd in a logarithmic scale. A regression line was fitted to these data with r = 0.74. Samples were processed in duplicates.

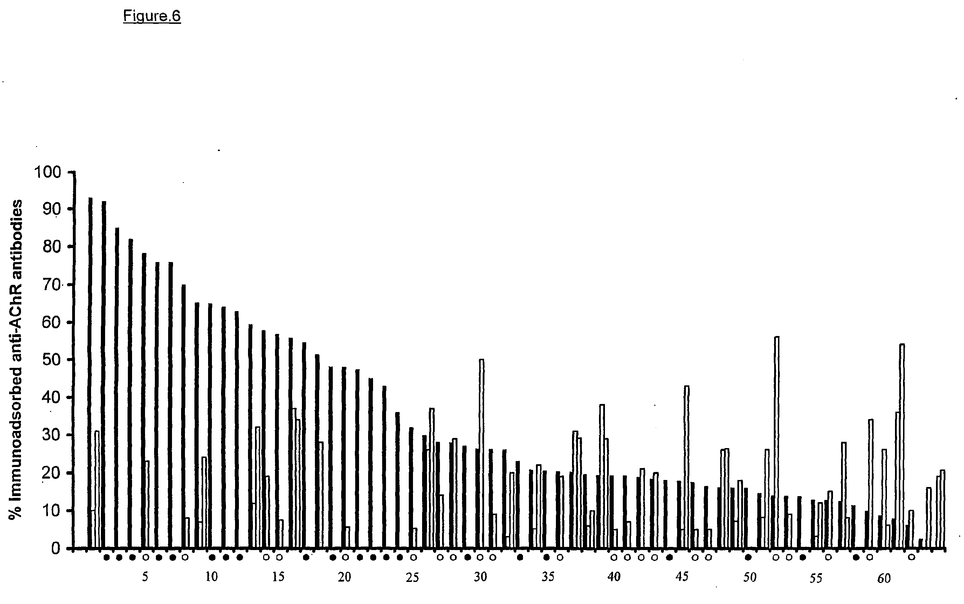

**Figure 6** illustrates the immunoadsorption of anti-AChR antibodies from MG sera using immobilized alpha1-210 (black bars), gamma1-218 (grey bars) and epsilon1-219 (blank bars). Each subunit has been expressed in the yeast *Pichia pastoris* and the purified proteins have been covalently immobilized on CNBr-Sepharose beads. 64 MG positive sera were assayed for binding to human AChR after incubation with alpha1-210 immobilized on CNBr-Sepharose (together with BSA) or with just BSA immobilized on the same insoluble matrix. 2.5ul of each MG patient serum were incubated with 0.2ug immobilized alpha1-210 mixed with BSA or the same amount of BSA at 4°C for 2-3h (in a total volume of 0.16 ml) followed by centrifugation. Samples of the supernatants, each containing an amount corresponding to 0.9 ul of the treated MG sera were subsequently used in a typical RIA MG diagnostic test (Lindstrom et al., Meth. Enzymol. 74, 432-460, 1981) with human AChR from TE671 cells. The percentage of immunoadsorption was estimated as described in the Example 3. Samples were processed in duplicates. Results shown (black bars) are an average of two experiments. Sera are sorted in decreasing percentage of immunoadsorption to immobilized alpha1-210.
   A subset of 50 sera was also tested with gamma1-218 immobilized on CNBr-Sepharose (gray bars) and a subset of 22 sera was screened against epsilon1-219 immobilized on CNBr-Sepharose (blank bars) as described in the Example 3. Sera which have not been tested for immunoadsorption with epsilon1-219 are indicated in the X axis with open circles and sera which have not been tested on both gamma1-218 and epsilon1-219 are indicated with filled circles.

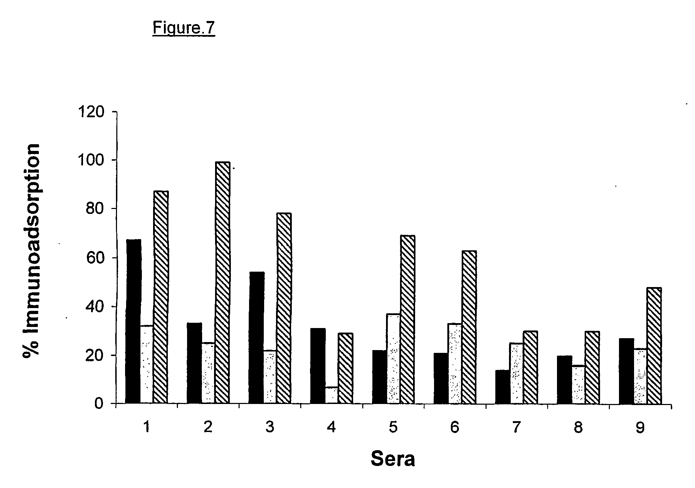

**Figure 7** illustrates the immunoadsorption of anti-AChR antibodies from MG sera against immobilized alpha1-210 (black bars), gamma1-218 (gray bars) and a mix of both immobilized alpha1-210 and gamma1-218 (hatched bars). 9 MG sera were assayed for binding to human AChR after incubation of 20 fmoles anti-AChR MG antibodies with alpha1-210 (1ug), or gamma1-218 (1ug) or alpha1-210/gamma1-218 mix (containing 1ug of each subunit) immobilized on CNBr-Sepharose. Incubation with just BSA immobilized to the same insoluble matrix served as a control to calculate the percent immunoadsorption against each of the subunits and the mix of both. After incubation with the Sepharose-immobilized proteins, sera were screened in a typical RIA experiment using TE671 AChR. The percentage of immunoadsorption was estimated as described in the Example 3. Samples were processed in duplicates. Results shown are an average of two experiments. The results clearly show that the combination of the two subunits has an additive effect on immunoadsorption.

### Detailed description of the invention

The N-terminal extracellular domains (or larger pieces containing said domains, or smaller pieces within said domains) of all five subunits of the human muscle AChR, are expressed preferably in eucaryotic systems (muscle AChR alpha and beta subunits are also named in the literature alpha1 and beta1 subunits respectively). As examples, we used the yeast *Pichia pastoris* expression system (Example 1 and Fig. 1A), the Semliki Forest Virus (SFV) expression system (Example 4), and the baculovirus expression system. In addition, alpha1-208 was also expressed in E. coli according to our previous publication (Tsouloufis et al., Int. Immunol. 12, 1255-1265, 2000).

The recombinant alpha subunit domain is expressed in a soluble form, and purified from the culture supernatant by affinity chromatography (Fig 1 B) or by ion exchange or gel filtration chromatography (Fig 3). The molecular weight of the product is estimated as higher than that predicted from the amino acid sequence, this difference being due to glycosylation of the molecule at residue 141, since enzymatic deglycosylation using peptide N-glycosidase F results in a reduction in the apparent molecular weight (Fig. 1C). These results show that, like the native receptor alpha subunit, alpha1-210 is glycosylated (Fujita et al, Science 231, 1284-1287, 1986).

An indication of the quality of conformation specifically of the human alpha subunit fragment may be obtained from studying binding of ¹²⁵I-alpha-BTX to the protein. Although E. coli expressed Torpedo extracellular alpha subunit fragment, especially after refolding, binds well ¹²⁵I-alpha-BTX, this does not happen with similarly expressed human alpha subunit fragments, even after refolding procedures (Tsouloufis et al., Int. Immunol. 12, 1255-1265, 2000). To study the binding of ¹²⁵I-alpha-BTX to alpha1-210 as an indication of correct protein folding, we use a filter assay experiment (Fig 2A). A high affinity for alpha-BTX is obtained. However, when the protein is deglycosylated by an appropriate enzyme, ¹²⁵I-alpha-BTX ability is practically totally lost (Fig 2C).

An important parameter of the quality of conformation of the protein is its water-solubility. As described in Example 1 and Fig 3, gel filtration analysis shows that the purified protein is water-soluble and migrates as a monomer.

Before evaluating the binding of MG sera to the polypeptides, the binding of several conformation-dependent anti-MIR and non-MIR monoclonal antibodies (mAbs) to alpha1-210 could be tested. As it is shown in Fig. 4 and described in Example 1, all tested mAbs (against the extracellular part of the alpha subunit) are able to bind to the protein, further suggesting that the molecule assumes the correct conformation. There are not many monoclonal antibodies specific for the extracellular domains of the non-alpha human AChR subunits, but we could test the gamma subunit domain with two conformation-dependent monoclonal antibodies, mAbs 66 and 67. Both monoclonal antibodies were shown to bind well to said polypeptide.

Other expression systems may be also used, as for example the Semliki Forest Virus expression system described in the Example 4 and the baculovirus expression system which we are also using. Similarly, the extracellular parts of the non-alpha muscle AChR subunits are expressed as for example is described in the Example 2 for the extracellular domains of the beta, gamma, delta and epsilon subunits expressed in the *Pichia pastoris* system.

Several modifications in the constructs and expressions may be introduced in order to improve the yield or the conformation of the polypeptides. For example, in some constructs we have introduced the FLAG tag at the N-terminal (in the presence or absence of the 6His tag), variations in the size of the polypeptides (for example, we have expressed in the Pichia pastoris system the human alpha1-80, 41-119, 1-119 and 1-211 polypeptides), mutations to replace the free cysteins of gamma and delta subunits by other amino acids (preferred alanine), and substitutions of the hydrophobic loops, of the various subunits, corresponding to residues approximately 128-142 of the alpha-subunit, with hydrophilic counterparts, preferably suggested from the corresponding sequence of the ACh binding protein (AChBP) (Brejk et al., Nature, 411, 269-276, 2001) as we have performed for the gamma subunit polypeptide. Finally, the form of the alpha domain that includes the P3A exon may be used in the place of the plain alpha domain. The provided protein yields in the examples refer to yeast cultures in flasks. Use of fermentors allows dramatic increase of the production efficiency.

Since the antibody- and ligand-binding experiments indicate that the recombinant proteins assume the correct conformation, one can study the binding of anti-AChR autoantibodies from MG sera to the recombinant proteins, since these antibodies are, in general, highly conformation-dependent (Tzartos et al. Immunol. Rev. 163, 89-120, 1998). Example 1 and Fig. 5 describe the screening of 70 human sera (50 MG and 20 healthy controls). Their binding to the recombinant protein is compared to that on a solubilized hybrid AChR, containing human alpha subunit and *Torpedo* beta, gamma and delta subunits; since MG sera essentially do not bind to *Torpedo* AChR (Loutrari et al., Clin. Exp. Immunol. 109, 538-546 1997), their binding to the hybrid molecule represents binding to the alpha subunit in the hybrid AChR molecule. By this procedure we are able to compare antibody binding to the alpha subunit inserted in the intact AChR molecule and to the isolated recombinant alpha subunit fragment. Most sera are found to recognize the recombinant protein and this binding seems proportional to their binding to the alpha subunit on the human/Torpedo AChR hybrid, suggesting that a major fraction of the anti-alpha autoantibodies in MG patients bind to recombinant alpha1-210. This exact kind of experiment could not be performed with the polypeptides of the other subunits (non-alpha) since said subunits do not bind alpha-BTX. We therefore proceeded directly to the permanent immobilization of said subunit domains and their use as immunoadsorbents, as described below.

The purified polypeptides are permanently (preferably covalently) immobilized on an insoluble matrix, selected from several suitable matrixes known in the art, including agaroses (for example CNBr-Sepharose), celluloses, porous glass, silica, resins, synthetic matrixes including acrylamide derivatives, methacrylamide derivatives or polystyrene derivatives etc, in various forms including beads, fibrous form, sheets or hollow fibers, with spacer arms (like for example NHS-activated Sepharose 4 Fast Flow) or without (like for example CNBr-activated Sepharose 4B) by approaches known in the art for immobilization of other ligands. We covalently immobilized the AChR polypeptides on CNBr-activated Sepharose 4B (Amersham) in most of the experiments, but also on Epoxy-activated agarose (Sigma) with similar efficiency, and on NHS-activated Sepharose 4 Fast Flow (Amersham) and ECH-Sepharose 4B (Amersham) with variable efficiency. Said permanently immobilized polypeptides of all five subunits were subsequently used for the immunoadsorption of anti-AChR antibodies from MG sera and plasma. In the Example 3 and Fig. 6 (black bars) is shown that the immobilized alpha domain is able to immunoadsorb more than 60% of the anti-AChR antibodies in several MG sera. The similarly immobilized extracellular alpha domain expressed in E. coli (alpha1-208) and refolded according to Tsouloufis et al. (Int. Immunol. 12, 1255-1265, 2000) was also used as immunoadsorbent of the same MG sera; its absorbing efficiency was significant but much lower.

Fig. 6 (gray and blank bars) also shows that the immobilized gamma and delta subunit domains are also able to immunoadsorb large fractions of anti-AChR antibodies from several MG sera. The other subunit domains (beta and delta) when similarly immobilized on Sepharose-CNBr could also immunoadsorb similar antibody fractions (not shown).

Finally, extracellular polypeptides from different subunits, permanently immobilized on solid carriers, were mixed and used as immunoadsorbents. Said mixed immunoadsorbents eliminated increased percentages of the anti-AChR antibodies approximately equal to the sum of the immunoadsorptions achieved by the individual subunit immunoadsorbents. Fig. 7 compares the immunoadsorbing capacity of the combination of the immobilized alpha and gamma subunit extracellular domains with the capacity of said immunoadsorbents when used separately. It is shown that the effect of their combined use was approximately equal to the sum of their effects when used separately. Thus, although any single immobilized subunit polypeptide may be capable of eliminating large percentages of anti-AChR antibodies from a few MG patients, the combined use of said polypeptides is highly preferable for the majority of the MG patients.

The produced immunoadsorbents can be easily packed into appropriate columns and be used in therapeutic apheresis systems, like those used in Ig apheresis systems known in the art, for the tentative MG therapy.

Expression of larger fragments or intact subunits containing the extracellular domains, or smaller fragments containing large parts of the extracellular domains of the AChR subunits, will apparently behave similarly and are obviously covered by this patent application. Use of similar subunits or domains from AChRs of mammalian species by applying the above approaches should be possible but they would absorb much lower fractions of MG antibodies (due to the low cross-reactivity of the MG antibodies described above) and therefore they would not be satisfactory for MG treatment. Only subunits or domains from very similar muscle AChRs, for example from primate species, could possibly be sufficiently efficient and are obviously covered by this patent application. :Below are provided some examples to prove the feasibility of the approach.

### Example 1. Expression of extracellular domain of human AChR alpha subunit in yeast Pichia pastoris, its purification and characterization.

The N-terminal extracellular domain of the human muscle nicotinic AChR alpha subunit (alpha1-210) was expressed in yeast *Pichia pastoris.* The yeast expression system allows post-translational modifications including glycosylation, thus resulting in correct protein folding, while the ease of manipulation, short doubling time, and high yield of protein expression are similar to those using bacteria. We used the methylotropic yeast strain, *Pichia pastoris,* as it has a strong inducible promoter and it is less prone to hyperglycosylation than *Saccharomyces cerevisiae*.

Alpha 1-210 was enzymatically amplified by PCR using a full-length human muscle alpha cDNA clone. The upstream (5'-GCTGGCCTCGTCGAATT-CTCCGAACATG-3') and downstream (5'-GATGAAGTAGAGGTCTAGACGCTGCATGACG-3') primers were constructed to contain EcoRI and XbaI restriction sites (underlined). Using the appropriate restriction endonucleases, the purified cDNA fragment was subcloned into the expression vector pPICZalphaA (Invitrogen San Diego, CA) so that the recombinant fragment was led by a signal peptide alpha-factor under the transcriptional control of the AOX promoter, which is induced by methanol, while at the C terminal end it was fused to a sequence encoding the c-myc epitope and polyhistidine (6xHis) tag (Fig. 1A). The resulting construct was linearized using Pmel and transformed into the *Pichia pastoris* host strain GS115 by electroporation (BioRad GenePulser). Similar were the constructs used for the alpha1-80, 41-119 and 1-119, 1-211 polypeptides. The transformed cells were plated on YPDS (1% yeast extract, 2% peptone, 2% dextrose, and 1M sorbitol) plus zeocin (100 ug/ml) and incubated at 30°C for 3 days.

Single colonies of the transformants were initially inoculated into 4 ml of BMGY (1% yeast extract, 2% peptone, 100 mM potassium phosphate, pH 6.0, 1.34% YNB, 4 X 10⁻⁵% biotin, and 1 % glycerol). After 16-20 h at 30°C, the cells were resuspended in 4 ml of BMMY (identical to BMGY, except that the glycerol was replaced by 0.5% methanol) to induce expression. After induction for 3 days with daily addition of methanol (0.5% v/v), the culture supernatants were tested for expression of alpha1-210 by dot-blot analysis using the anti-myc 9E.10 mAb (ATCC). The clone with the highest protein yield was used for large-scale protein expression.

The culture supernatant was concentrated using a Minitan Ultrafiltration System (Millipore) and dialyzed against PBS, pH 8, then alpha1-210 was purified using NiNTA metal affinity chromatography (Qiagen) according to the manufacturer's protocol, the recombinant protein being eluted under native conditions using increasing imidazole concentrations (40, 70, and 100 mM). The eluates were analyzed by 12% SDS-PAGE followed by Coomassie Brilliant Blue staining (Fig. 1 B) or Western blot analysis using an anti-alpha subunit mAb (mAb198) or the anti-myc 9E.10 mAb. The protein concentration was determined using the Bradford method (BioRad) and the yield of purified alpha1-210 was estimated to 0.2-0.3 mg/l. The molecular weight of the product was estimated as 34 kD, higher than that predicted from the amino acid sequence (27 kD); this difference was shown to be due to glycosylation of the molecule at residue 141, since enzymatic deglycosylation using peptide N-glycosidase F (PNGase F, New England Biolabs) resulted in a reduction in the apparent molecular weight to about 27 kD (Fig. 1 C). These results show that, like the native AChR alpha subunit, alpha1-210 is glycosylated.

### High-affinity binding of ¹²⁵I-alpha-BTX to purified alpha1-210.

To study the binding of alpha-BTX to alpha1-210 as an indication of correct protein folding, we tested both crude culture supernatants and the purified protein in "filter assay" experiments: Fifty nanograms of purified alpha1-210 or 20-40 ml of culture supernatant was incubated at 4°C for 3 h with various concentrations of ¹²⁵1-alpha-BTX (specific activity 800,000 cpm/pmole) in a final volume of 50 ml of PBS buffer, pH 7.5, 0.2% BSA. The samples were then diluted with 1 ml of 0.5% Triton X-100 in 20 mM Tris buffer, pH 7.5 (Triton buffer) and immediately filtered through two Whatman DE81 filters prewashed with Triton buffer. The filters were then washed twice with 1 ml of Triton buffer and the bound radioactivity measured on a gamma-counter. Samples without alpha1-210 were used to measure non-specific binding. Specific binding of alpha-BTX was also studied in competition experiments by addition of various amounts of unlabeled alpha-BTX to the sample.

As shown in Fig. 2A, alpha1-210 bound alpha-BTX with a Kd, estimated by Scatchard analysis, of 5.1 ± 2.4 nM, which is only one order of magnitude lower than that of the native human AChR.

The binding of ¹²⁵I-alpha-BTX to alpha1-210 was shown to be specific using various concentrations of unlabeled alpha-BTX in competition assays (Fig. 2B). Unlabeled toxin inhibited the binding of labeled alpha-BTX even at low concentrations. Moreover, the results were consistent with the Kd estimated from the Scatchard plot, since 50% inhibition of ¹²⁵I-alpha-BTX binding was seen using unlabeled toxin at a concentration of 5 nM. Alpha-BTX binding was markedly impaired by *in vitro* deglycosylation of alpha1-210 (Fig. 2C)

### FPLC analysis.

The solubility and size of the recombinant protein were studied by FPLC on a Superose-12 column (Pharmacia - Biotech) using PBS buffer, pH 7.5, at a flow rate of 0.5 ml/min, followed by assay of the fractions for ¹²⁵I-alpha-BTX binding. As shown in. Fig. 3, the protein migrated as a monomer with a molecular weight consistent with that estimated by SDS-PAGE.

### mAb binding to alpha1-210.

The binding to alpha1-210 of several anti-MIR mAbs derived from rats immunized with intact AChR from either human muscle (mAb 190, 192, 185, 198, and 202) or Electrophorus electricus electric organ (mAb35) was tested by RIA. This study would be expected to provide useful information about the protein conformation, since some of the mAbs are known to bind exclusively to the native non-denatured AChR (mAb192, 190, and 35), while the binding of others is only partially conformation-dependent (mAb198, 195, and 202). In addition, we tested the binding of mAb64, an anti-alpha mAb not directed against the MIR. mAb25, which does not bind to mammalian AChR was used as a negative control.

Alpha1-210 (50 ng) was labeled by incubation for 3 h at 4°C with ¹²⁵I-alpha-BTX (50,000 cpm) in a total volume of 40 ul, then 10 ul of diluted mAb (1:200) containing 0.1 ul of normal rat serum as carrier was added and the samples incubated at 4°C for 15-18 h. Immune complexes were then precipitated by addition of 10 ul of rabbit anti-rat immunoglobulin serum and incubation for 1.5 h at 4°C, followed by centrifugation (4,000 rpm at 4°C for 10 min). The precipitates were washed three times with PBS-0.5% Triton-X 100 and the precipitated radioactivity counted.

The results of these RIA experiments are shown in Fig. 4 where, with the exception of the negative control (mAb25), all the mAbs tested (dilution 1:200) bound to the recombinant protein in RIA experiments. Overall, the fact that all tested mAbs were able to bind to recombinant alpha1-210 further suggests that the molecule assumes the correct conformation.

### Binding of MG patients' autoantibodies to alpha 1-210.

As the antibody- and ligand-binding experiments indicated that the recombinant protein assumed the correct conformation, we then studied the binding of anti-AChR autoantibodies from MG sera to alpha1-210, since these antibodies are, in general, highly conformation-dependent. 70 human sera were screened: 50 MG and 20 healthy controls (5ul of human sera/sample). In order to determine the anti-alpha subunit specificities of the MG antisera, we analyzed, in parallel, their capacity to bind to recombinant alpha1-210 (0.4pmole labeled with 150,000cpm ¹²⁵I-alpha-BTX) and to a solubilized hybrid AChR molecule (HaTbgd, formed by human alpha subunit and Torpedo beta, gamma and delta subunits) (17fmole labeled with 50,000cpm ¹²⁵I-alpha-BTX); since MG sera essentially do not bind to *Torpedo* AChR, their binding to the hybrid molecule represents binding to the alpha subunit in the hybrid AChR molecule, and we were therefore able to compare antibody binding to the alpha subunit inserted in the intact AChR molecule and to the isolated recombinant alpha subunit fragment. Thirty-six of the 50 MG sera (72%) were found to have high titers of anti-alpha autoantibodies (i.e. they immunoprecipitated an amount of radiolabeled hybrid AChR molecule greater than the mean of the controls + 3 standard deviations of that immunoprecipitated by healthy control sera) and 24 of these 36 (66%) recognized alpha1-210 (i.e. they immunoprecipitated an amount of radiolabeled recombinant molecule greater than the mean of the controls + 3 standard deviations of that immunoprecipitated by healthy control sera). Interestingly, there was a strong linear correlation between the binding of human sera to ¹²⁵I-alpha-BTX-labeled alpha1-210 and to the hybrid HaTbgd AChR (r=0.74) (Fig. 5), suggesting that a major fraction of the anti-alpha autoantibodies in MG patients bind to recombinant alpha1-210.

### Example 2. Expression of the extracellular domains of beta, gamma, delta and epsilon subunits their purification and characterization.

The DNA encoding the N-terminal extracellular domains of the human muscle nicotinic AChR beta, gamma and epsilon subunits was enzymatically amplified using PCR, on templates pcDNA-beta, pcDNA-gamma and pcDNA-epsilon respectively (plasmids carrying the whole cDNA of the gamma and epsilon subunits cloned under the CMV promoter) (plasmids provided by Dr Beeson, University of Oxford). PCR was performed using primers as follows. For beta subunit, upstream primer 5'-GCGGAATTCTCGGAGGCGGAGGG-TCGAC-3' was combined with downstream primer 5'-ATAGTTTAGCGGCCGCTCAATGGTGATGGTGATGGTGCTTGCGGCG-GATGATGG-3' on template pcDNA-beta. For gamma subunit, upstream primer 5'-GGTGTAGAATTCCGGAACCAGGAGGAGCGC-3' was used with downstream primer 5'-ATAGTTTAGCGGCCGCTTAGTGATGGTGATGGTGATGCTTGCGCTG-GATGAGCAGG-3'. For epsilon subunit, upstream primer 5'-GGTGTAGAATTCAAGAACGAGGACTGCG-3' was combined with downstream primer 5'-ATAGTTTAGCGGCCGCTTAGTGATGGTGATGGTGATGCTTCCGGC-GGATGATGAGCGAG-3'. All upstream primers were designed to contain an *Eco*RI site and the downstream primers were designed to contain a *Not*I site (these sequences are underlined in the shown primer sequences). All downstream primers contain a sequence which encodes for a six-histidine aminoacid tag to facilitate the purification of the expressed products (Fig. 1). For the expression of the subunits in the yeast *Pichia pastoris,* the obtained PCR fragments were digested with *Eco*RI and *Not*I enzymes, purified and cloned into the pPIC9 (Invitrogen, San Diego, CA) or pPIC9/FLAG vectors. The pic9/FLAG is a modification of the pPIC9 vector. This modified pPIC9/FLAG vector contains the FLAG amino acid sequence (DYKDDDDK) immediately after the cleavage site of the secretion signal, which has been shown to increase solubility of the expressed polypeptide in *Pichia,* probably due to the highly hydrophilic nature of the amino acids. The modification was performed by cloning the oligo 5'-GTAGATTACAAGGATGACGATGACAAAG-3' between the unique *SnaB*I and *Eco*RI sites of the pPIC9 vector. This allows the subsequent cloning of any PCR product with an *Eco*RI site. The resulting plasmid was named pPIC9/FLAG.

The constructs (pPIC9/FLAG-beta1-222, pPIC9/FLAG-gamma1-218 and pPIC9/FLAG-epsilon1-219) were linearized using *Sac*I and transformed into competent *Pichia pastoris* strain GS115 cells by electroporation (Biorad GenePulser). The transformed cells were plated on RDB plates (1 M sorbitol, 2% dextrose, 1.34% YNB, 4x10⁻⁵% biotin, 0.005% of each of L-glutamic acid, L-methionine, L-lysine, L-leucine and L-isoleucine) plus ampicillin (100ug/ml). Single colonies of the transformants were initially inoculated into 2 ml of BMGY medium, grown overnight to an OD600 of approximately 2-6 and subsequently transferred to BMMY medium to induce the expression of the cloned gene. After induction for 3 days with daily addition of methanol (0.5% v/v), as described above, the culture supernatants were tested for expression by dot-blot analysis using the M2 anti-FLAG antibody (Sigma). For gamma1-218, an extra antibody was used, mAb 67 (conformation-dependent monoclonal antibody against gamma subunit). The best clone of each construct was picked for large-scale expression.

Large-scale expression of the products was performed by the inoculation of a single colony overnight in 5 ml BMGY medium. The following day a very small amount (100 microliters) of the culture was diluted in 1 It of BMGY medium and allowed to grow overnight again to an OD600=2-6, at which point the cells were collected and resuspended in BMMY medium at an OD600 of 1. Expression was allowed to proceed for two days (methanol was added to each culture daily). The culture supernatant was collected by centrifugation and the proteins were precipitated by the ammonium sulphate method. The precipitated proteins were obtained by centrifugation at 6000rpm (Sorvall RC5R) for 30 min and resuspension of the pellet in distilled water. Extensive dialysis of the protein solution followed in 100 mM phosphate buffer pH8.0-0.5M NaCl, before binding of the protein to pre-equilibrated NiNTA agarose (QIAGEN) for 2h at 4°C. Protein was eluted under native conditions in phosphate buffer containing 100mM imidazole. Protein fractions were analyzed on 12% SDS-PAGE followed by Coomassie staining and also by Western Blotting using anti-FLAG antibody. Protein concentration was consistently estimated to be around 0.4-0.9 mg per liter of culture for all three beta, gamma and epsilon subunit domains. The molecular mass of all protein bands for beta, gamma and epsilon were estimated to be 32-34kDa. This results from the glycosylation of the products in *Pichia.* Indeed, deglycosylation of the products using PNGase F (New England Biolabs, MA) resulted in the decrease in their molecular mass to ∼28 kDa (predicted from the amino acid sequence).

The N-terminal extracellular domain of the human muscle AChR delta subunit (delta1-224) was enzymatically amplified by RT-PCR using total RNA extracted from TE671 cells using the primers: 5'-GTGTGGCAGCGAATTCCTGAACGAG-3' (upstream for delta subunit) and 5'-GATGTAGAATTCTCACTTGCGGCGG-3' (downstream for delta subunit). The primers were constructed to contain EcoRI restriction sites (underlined). Using the appropriate restriction endonucleases, the purified cDNA fragment was subcloned into the expression vector pPIC9, so that the recombinant fragment was led by a signal peptide alpha-factor under the transcriptional control of the AOX promoter, which is induced by methanol, while at the C terminal end there was no sequence encoding the c-myc epitope or the polyhistidine (6xHis) tag. The resulting construct was linearized using Stul and transformed into the *Pichia pastoris* host strain GS115 or into the *Pichia pastoris* clone that expressed alpha1-210 (example 1) in order to express delta1-224 alone or coexpressed together with alpha1-210 respectively. The transformed cells were plated on YPDS plus ampicillin (50ug/ml). After induction for 3 days with daily addition of methanol (0.5% v/v), as described above, the culture supernatants were tested for expression of delta1-224 by dot-blot analysis using the anti-FLAG M2 monoclonal antibody (Sigma). The clone with the highest protein yield was used for large-scale protein expression. The culture supernatant was precipitated using ammonium sulfate and dialyzed against TBS, pH 8, then delta1-224 was purified using Anti-FLAG M2 affinity gel (Sigma) according to the manufacturer's protocol, the recombinant protein being eluted under native conditions by competition with FLAG peptide (Sigma). The eluates were analyzed by 12% SDS-PAGE followed by Coomassie Brilliant Blue staining or Western blot analysis using anti-FLAG M2 monoclonal antibody. The yield of purified delta1-224 was estimated to 0.1-0.5 mg/l. The recombinant protein appeared as three bands of different molecular weight, which after in vitro deglycosylation of the product (as described above) migrated to the predicted from the amino acid sequence (26 kD). These results show that, like the native receptor delta subunit, delta1-224 is glycosylated. Delta1-224 was studied together with alpha1-210 in ¹²⁵I-alpha-BTX binding experiments. Results indicated a temporary weak interaction between the recombinant subunit domains alpha1-210 and delta1-224.

### Example 3. Permanent immobilization of the recombinant proteins on insoluble material and their use for immunoadsorption of MG antibodies.

*Immunoadsorption of MG autoantibodies by immobilized alpha1-210*. 0.1 mg alpha1-210 mixed with 1.4 mg BSA was covalently bound to 0.5gr CNBr-Sepharose according to manufacturer's protocol (Amersham). BSA was simply added to increase the protein/Sepharose ratio because the ratio of recombinant protein to Sepharose beads used would otherwise be too small; BSA is not necessary for larger scale experiments. In parallel, 1.5mg BSA was bound to the same amount of CNBr-Sepharose under the same conditions as control. 2.5ul MG patient sera were incubated with 0.2ug immobilized alpha1-210 mixed with BSA, or the same amount of immobilized BSA alone, at 4°C for 2-3h (in a total volume of 0.16 ml) followed by centrifugation for bead precipitation. Samples of the supernatants, each containing an amount corresponding to 0.9 ul of these treated MG sera were subsequently used in a typical RIA MG diagnostic test (Lindstrom et al., Meth. Enzymol. 74, 432-460, 1981) with human AChR from TE671 cells. The percentage of immunoadsorption was estimated as the ratio between the reduction in immunoprecipitated radiolabeled human AChR after incubation with alpha1-210 and the immunoprecipitated radiolabeled human AChR without incubation with alpha1-210 (incubation with plain immobilized BSA). 64 MG positive sera were assayed for binding to human AChR after incubation with immobilized alpha1-210 or BSA. As shown in Fig. 6, in 13 of the 64 (20%) MG sera more than 60% of the anti-AChR antibodies were found to be eliminated, while in another 24 sera (37%) the immunoadsorption was between 20-60%. We also tested immunoadsorption of the same set of MG sera by incubating 0.2 ug immobilized alpha1-210 with a fixed amount of anti-AChR antibodies (20 fmoles in variable volumes of MG sera depending on their titer) rather than a fixed serum volume. These experiments gave identical with the above immunoadsorption results.

The above described ratio of immobilized polypeptide versus MG antibodies is high, but it was found not necessary. Titration experiments showed that 1 ug of immobilized alpha1-210 is able to eliminate the anti-alpha antibodies from at least 0.25ml MG serum of titer 5nM (i.e. 1250 fmoles).

Finally, alpha1-210 (both in soluble and in immobilized form) was found resistant to proteolysis by the presence of human plasma, as even 15-18h incubation of the recombinant protein (¹²⁵I-labeled or unlabeled) with human plasma at 30°C did not have any effect on the integrity of the molecule (judged by SDS-PAGE) and radiolabeling experiments.

*Immunoadsorption of MG autoantibodies by immobilized gamma1-218 or epsilon1-219 domains*. 0.1 mg□ of purified gamma1-218 or epsilon 1-219 mixed with 1.4mg BSA was covalently immobilized on 1 gr of CNBr-Sepharose according to manufacturer's protocol. In parallel, an identical column was prepared with 1.5 mg BSA, to serve as control. Variable amounts of MG patient sera containing 20 fmoles of anti-AChR antibodies were incubated with 1ug immobilized gamma1-218 mixed with BSA or the same amount of BSA at 4°C for 2-3h followed by centrifugation. Samples of the supernatants, each containing an amount corresponding to 6 fmoles of preadsorbed anti-AChR antibody, were subsequently tested in a typical RIA MG diagnostic test as above. Sera which had been incubated with gamma1-218 were screened with the human AChR from TE671 cells (which contains alpha, beta, gamma and delta subunits) whereas sera immunoadsorbed with epsilon1-219 were screened with human AChR from TE671-epsilon cells(which contains alpha, beta, gamma and epsilon subunits) (Beeson et al., Neurology, 47,1552-1555, 1996). The percentage of immunoadsorption was estimated as above. Several MG sera from the group of the sera tested with immobilized alpha1-210 were assayed for binding to human AChR after incubation with immobilized gamma1-218 (43 sera) or epsilon1-219 (22 sera) or BSA only. Incubation with gamma1-218 led to a 29-56% reduction for 16% of the tested sera (7 sera out of 43), whereas it caused a 15%-30% reduction in another 28% of the tested sera (12 sera) (Figure 6, gray bars). Other tested MG sera reached up to 68% immunoadsorption by gamma1-218 (not shown). Titration experiments showed that the used amounts of immobilized gamma domain were in excess and are capable of immunoadsorbing the antibodies from larger MG volumes. Blank bars in Figure 6 show the immunoadsorption of 22 MG sera by the immobilized epsilon1-219 led to a 30-54% reduction in the antibodies in 27 % of the screened sera (6/22) and 15%-30% reduction in another 45% of the sera (10/22). Therefore, the results show that the expressed gamma and epsilon subunit domains can immunoadsorb considerable amounts of the MG anti-AChR antibodies.

Experiments with radiolabelled gamma1-218 suggested that the protein is stable after incubation with normal human serum for at least 6 hours at room temperature, suggesting that the protein is not degraded or proteolyzed by serum enzymes.

*Immunoadsorption of MG autoantibodies by combinations of immobilized subunit domains*. To evaluate whether the combination of more than one immobilized subunit domains would result in an increase in the immunoabsorption of pathogenic antibodies (additive effect), we performed experiments where CNBr-Sepharose carrying alpha1-210 was mixed with CNBr-Sepharose carrying gamma1-218. A set of nine MG sera (in addition to negative controls) were incubated (using the standard conditions) either with 1 ug immobilized alpha1-210, or 1ug immobilized gamma1-218 or the mix of the two subunits (containing 1ug of each subunit). The results showed that the incubation of the sera with the mix led to an additive effect on immunoadsorption (Figure 7). For example, serum no. 3 which gave 54% immunoadsorption on alpha1-210 and 22% on gamma1-218, gave a total of 78% against the mix of both proteins. These results suggest that the combination of all five subunits should lead to the specific removal of the majority of the pathogenic antibodies.

### Example 4. Expression of extracellular domain of human AChR alpha subunit in human cell cultures by the Semliki Forest Virus system (SFV) expression system and its further study and use.

The replication deficient-Semliki Forest Virus expression system is based on two plasmid vectors containing Semliki Forest virus cDNA. The pSFV-1 vector contains the region required for the production of the nsp1-4 replicase complex. A strong viral promoter is located at the 3'-end of the *nsp4* gene followed by a polylinker for the introduction of foreign genes. The pSFV helper vector encodes the viral structural genes (Liljeström and Garoff, 1991). Plasmid pSFV-1 containing the gene of interest can be used alone or together with the helper plasmid. In the former case (recombinant pSFV plasmid used alone) one only gets expression of the cloned gene whereas in the latter, one gets production of a recombinant viral stock which can be subsequently used to infect a larger number of host cells.

The DNA fragment encoding the extracellular domain of the human alpha AChR (alpha 1-210) was amplified by PCR using upstream primer 5'-GCGGCCGCATGAAGGTTCTGTGGGC-TGCGTTGCTGGTCACATTCCTGGCAGGATGCCAGGCCTCCGAACATGAGACC CG-3' and downstream primer 5'-CCGAGCCTCGAGT-CAATGATGATGATGATGATGGTCGACG-3', on template pTEP1-1. The primers provide the *Not*I and *Xho*I sites (underlined) for further manipulation of the DNA. Upstream primer also provides the leader sequence of the human apolipoprotein E (ApoE); this sequence encodes the recognizable tag on ApoE, which allows for extracellular secretion of the protein and therefore it might also allow secretion of our protein. Dowstream primer encodes six additional histidine residues prior to the termination signal; the extra histidines will serve as a purification tool by use of nickel-agarose affinity chromatography. The obtained PCR product was digested using enzymes *Not*I and *Xho*I, filled in with the Klenow fragment of DNA polymerase I and cloned into Smal site of the SFV-1 vector DNA.

Prior to the actual experiment with the SFV vector, the PCR product was additionally cloned into a commercial eucaryotic expression vector (pcDNA3.0+, Invitrogen) and the recombinant plasmid was transfected into human HEK293 cells. Approximately 2x10⁶ transfected cells were allowed to express the protein of interest. Cells and medium supernatant were collected after two days. Cells were washed in cocktail buffer (1x PBS pH7.4 with PMSF 1 mM, aprotinin 5units/ml, leupeptin 5micrograms/ml, iodoacetamide 1 mM). The medium supernatant (12ml) was concentrated 5 times to a final volume of 2.5 ml and dialysed against PBS buffer at 4°C. To extract the receptor, the cell pellet was resuspended in 0.5ml of cocktail-2% Triton-X100 and the mixture was left at 4°C with mild agitation. The mixture was subsequently centrifuged at 15000g for 15 min and the supernatant was tested for ¹²⁵I-alpha-BTX binding. The filter assay technique (as described in Example 1) was used to test whether the expressed protein was secreted in the medium supernatant or retained within the cell (in either the cytoplasm or the membrane). The results indicated that good expression of functional human extracellular domain of alpha subunit is achieved, but that the protein is present intracellularly.

Having established that the cloned product is functional, the recombinant SFV plasmid is linearized at *Spe*I site, purified and used in an *in vitro* mRNA synthesis using the mMESSAGE mMACHINE kit (Ambion Inc.) Approximately 20 micrograms of mRNA produced is obtained and used to electroporate 0.7 ml of 10⁷ cells/ml HEK293 cells. We only use the recombinant SFV-1 vector without the helper plasmid at this stage. Both the cells and the supernatant are collected two days post-electroporation. Cells are washed, disrupted by use of the above described cocktail-2% Triton X100 buffer and further treated as described above. The medium supernatant is collected, concentrated and dialyzed as described above. The results of filter assay experiments using the concentrated/dialyzed medium supernatant and the supernatant of the extracted cells showed that the protein is expressed at satisfactory levels. The protein appears to be functional and displaying a good ability to bind ¹²⁵I-alpha-BTX.

## Claims

1. Use of a combination of recombinant N-terminal extracellular domains of the alpha, beta, gamma, delta and epsilon subunits of the human muscle nicotinic acetylcholine receptor (AChR) in an ex-vivo method of immunoadsorption of anti-AchR antibodies of myasthenia gravis (MG) patients.

2. A use as claimed in claim 1 wherein the recombinant domains are mutant forms which include substitutions of free cysteine by other amino acids and substitutions of the hydrophobic loops of the subunits corresponding to alpha 128-142 by more hydrophilic sequences, or the alpha domain containing the P3A exon, or comprise a FLAG tag at the N-terminal in the presence or absence of the 6His tag.

3. A use as claimed in any preceding claim wherein the recombinant domain of the alpha subunit is the N-terminal extracellular domain comprising amino acids 1-210.

4. A use as claimed in any preceding claim wherein the recombinant domain of the beta subunit is the N-terminal extracellular domain comprising amino acids 1-222.

5. A use as claimed in any preceding claim wherein the recombinant domain of the gamma subunit is the N-terminal extracellular domain comprising amino acids 1-218.

6. A use as claimed in any preceding claim wherein the recombinant domain of the delta subunit is the N-terminal extracellular domain comprising amino acids 1-224.

7. A use as claimed in any preceding claim wherein the recombinant domain of the epsilon subunit is the N-terminal extracellular domain comprising amino acids 1-219.

8. A use as claimed in any preceding claim wherein the recombinant domains are expressed in a eukaroytic expression system.

9. A use as claimed in claim 13 wherein the eukaryotic expression system is Pichia pastoris or SFV.

10. A use as claimed in any preceding claim wherein the recombinant domains are large sequences of more than approximately 70 amino acids long and, preferably, about 200 amino acids long.

11. A use as claimed in any preceding claim wherein the combination is achieved simultaneously or sequentially.

12. An insoluble carrier comprising a combination of recombinant domains of the human muscle nicotinic AChR subunits alpha, beta, gamma, delta and epsilon as defined in any one of claims 1 to 11, wherein the domains are covalently immobilized to the carrier matrix.

13. An insoluble carrier as claimed in claim 12 wherein the carrier matrix is selected from agaroses, such as CNBr-Sepharose, celluloses, porous glass, silica, resins, synthetic matrixes including acrylamide derivatives, methacrylamide derivatives or polystyrene derivatives.

14. A method for making an insoluble carrier for use in a method of immunoadsorption of anti-AChR antibodies comprising:
i) expressing a combination of recombinant domains as defined in any of claims 1 to 11;
ii) covalently imobilizing said purified domains onto an insoluble carrier matrix.

15. A method for *ex vivo* removal of anti-AChR antibodies from the blood of MG patients comprising incubating said blood with an insoluble carrier as claimed in any of claims 12 to 13.

## Patentansprüche

1. Verwendung einer Kombination von rekombinanten N-terminalen extrazellulären Domänen der Alpha-, Beta-, Gamma-, Delta- und Epsilon-Untereinheiten des humanen nikotinischen Acetylcholinrezeptors (AChR) aus Muskel in einem *ex vivo*-Verfahren zur Immunadsorption von Anti-AChR-Antikörpern von Patienten mit Myasthenia gravis (MG).

2. Verwendung nach Anspruch 1, wobei die rekombinanten Domänen mutierte Formen sind, die Substitutionen von freiem Cystein durch andere Aminosäuren und Substitutionen der hydrophoben Schleifen der Untereinheiten, die Alpha 128-142 entsprechen, durch hydrophilere Sequenzen enthalten oder die das P3A-Exon enthaltende Alpha-Domäne, oder die eine FLAG-Markierung am N-Terminus in Gegenwart oder Abwesenheit der 6His-Markierung umfassen.

3. Verwendung nach einem der vorangegangenen Ansprüche, wobei die rekombinante Domäne der Alpha-Untereinheit die N-terminale extrazelluläre Domäne ist, welche die Aminosäuren 1-210 umfaßt.

4. Verwendung nach einem der vorangegangenen Ansprüche, wobei die rekombinante Domäne der Beta-Untereinheit die N-terminale extrazelluläre Domäne ist, welche die Aminosäuren 1-222 umfaßt.

5. Verwendung nach einem der vorangegangenen Ansprüche, wobei die rekombinante Domäne der Gamma-Untereinheit die N-terminale extrazelluläre Domäne ist, welche die Aminosäuren 1-218 umfaßt.

6. Verwendung nach einem der vorangegangenen Ansprüche, wobei die rekombinante Domäne der Delta-Untereinheit die N-terminale extrazelluläre Domäne ist, welche die Aminosäuren 1-224 umfaßt.

7. Verwendung nach einem der vorangegangenen Ansprüche, wobei die rekombinante Domäne der Epsilon-Untereinheit die N-terminale extrazelluläre Domäne ist, welche die Aminosäuren 1-219 umfaßt.

8. Verwendung nach einem der vorangegangenen Ansprüche, wobei die rekombinanten Domänen in einem eukaryotischen Expressionssystem exprimiert sind.

9. Verwendung nach Anspruch 13, wobei das eukaryotische Expressionssystem Pichia pastoris oder SFV ist.

10. Verwendung nach einem der vorangegangenen Ansprüche, wobei die rekombinanten Domänen große Sequenzen mit einer Länge von mehr als ungefähr 70 Aminosäuren und bevorzugt einer Länge von etwa 200 Aminosäuren sind.

11. Verwendung nach einem der vorangegangenen Ansprüche, wobei die Kombination gleichzeitig oder nacheinander erhalten wird.

12. Unlöslicher Träger, welcher eine Kombination von rekombinanten Domänen der Alpha-, Beta-, Gamma-, Delta- und Epsilon-Untereinheiten des humanen nikotinischen AChR aus Muskel, wie in einem der Ansprüche 1 bis 11 definiert, umfaßt, wobei die Domänen auf der Trägermatrix kovalent immobilisiert sind.

13. Unlöslicher Träger nach Anspruch 12, wobei die Trägermatrix unter Agarosen, wie CNBr-Sepharose, Zellulosen, porösem Glas, Siliciumdioxid, Harzen, synthetischen Matrizes, einschließlich Acrylamidderivaten, Methacrylamidderivaten oder Polystyrenderivaten, ausgewählt ist.

14. Verfahren zum Herstellen eines unlöslichen Trägers zur Verwendung in einem Verfahren zur Immunadsorption von Anti-AChR-Antikörpern, welches folgendes umfaßt:
i) Exprimieren einer Kombination von rekombinanten Domänen, wie in einem der Ansprüche 1 bis 11 definiert,
ii) kovalentes Immobilisieren der gereinigten Domänen auf einer unlöslichen Trägermatrix.

15. Verfahren für das Entfernen von Anti-AChR-Antikörpern aus dem Blut von MG-Patienten *ex vivo*, welches das Inkubieren des Blutes mit einem unlöslichen Träger nach einem der Ansprüche 12 bis 13 umfaßt.

## Revendications

1. Utilisation d'une combinaison de domaines extracellulaires N-terminaux recombinants des sous-unités alpha, bêta, gamma, delta et epsilon du récepteur d'acétylcholine nicotinique du muscle humain (AChR) dans un procédé *ex-vivo* d'immuno-adsorption d'anticorps anti-AChR de patients souffrant de myasthénie grave (MG).

2. Utilisation selon la revendication 1, dans laquelle les domaines recombinants sont des formes mutantes qui comprennent des substitutions de cystéine libre par d'autres acides aminés et des substitutions des boucles hydrophobes des sous-unités correspondants à alpha 128-142 par des séquences plus hydrophiles, ou le domaine alpha contenant l'exon P3A, ou comprennent un marqueur FLAG en N-terminal, en présence ou en l'absence du marqueur 6His.

3. Utilisation selon l'une quelconque des revendications précédentes dans laquelle le domaine recombinant de la sous-unité alpha est le domaine extracellulaire N-terminal comprenant les acides aminés 1-210.

4. Utilisation selon l'une quelconque des revendications précédentes, dans laquelle le domaine recombinant de la sous-unité bêta est le domaine extracellulaire N-terminal comprenant les acides aminés 1-222.

5. Utilisation selon l'une quelconque des revendications précédentes, dans laquelle le domaine recombinant de la sous-unité gamma est le domaine extracellulaire N-terminal comprenant les acides aminés 1-218.

6. Utilisation selon l'une quelconque des revendications précédentes, dans laquelle le domaine recombinant de la sous-unité delta est le domaine extracellulaire N-terminal comprenant les acides aminés 1-224.

7. Utilisation selon l'une quelconque des revendications précédentes, dans laquelle le domaine recombinant de la sous-unité epsilon est le domaine extracellulaire N-terminal comprenant les acides aminés 1-219.

8. Utilisation selon l'une quelconque des revendications précédentes, dans laquelle les domaines recombinants sont exprimés dans un système d'expression eucaryote.

9. Utilisation selon la revendication 13, dans laquelle le système d'expression eucaryote est un Pichia pastoris ou SFV.

10. Utilisation selon l'une quelconque des revendications précédentes, dans laquelle les domaines recombinants sont de grandes séquences de plus d'environ 70 acides aminés et, de préférence, d'environ 200 acides aminés de long.

11. Utilisation selon l'une quelconque des revendications précédentes, dans laquelle la combinaison est obtenue simultanément ou séquentiellement.

12. Support insoluble comprenant une combinaison de domaines recombinants des sous-unités alpha, bêta, gamma, delta, et epsilon d'AcHr nicotinique de muscle humain, comme défini dans l'une quelconque des revendications 1 à 11, dans lequel les domaines sont immobilisés de manière covalente sur la matrice du support.

13. Support insoluble selon la revendication 12, dans lequel la matrice du support est choisie parmi les agaroses, comme la CNBr-Sépharose, les celluloses, le verre poreux, la silice, les résines, les matrices synthétiques comprenant des dérivés d'acrylamide, des dérivés de méthacrylamide ou des dérivés de polystyrène.

14. Procédé de réalisation d'un excipient insoluble à utiliser dans un procédé d'immuno-adsorption d'anticorps anti-AChR comprenant :
i) l'expression d'une combinaison de domaines recombinants comme défini dans l'une quelconque des revendications 1 à 11 ;
ii) l'immobilisation covalente desdits domaines purifiés sur une matrice de support insoluble.

15. Procédé d'élimination *ex vivo* d'anticorps anti-AChR du sang de patients souffrant de MG, comprenant l'incubation dudit sang avec un support insoluble selon l'une quelconque des revendications 12 à 13.
